# EUROPÄISCHE PATENTSCHRIFT

(11) **EP 3 542 863 B1**
(45) Veröffentlichungstag und Bekanntmachung des Hinweises auf die Patenterteilung: **17.03.2021**
(21) Anmeldenummer: 18176225.3
(22) Anmeldetag: 06.06.2018
(51) Int. Cl.: A61Q 5/00, A61K 8/92

(54) **HAARÖL UND SEINE VERWENDUNG**
HAIR OIL AND ITS USE
HUILE POUR LES CHEVEUX ET SON UTILISATION

(30) Priorität: 21.03.2018 EP 18163104
(43) Veröffentlichungstag der Anmeldung: 25.09.2019
(73) Patentinhaber: Miftari, Filiz, 46282 Dorsten (DE)
(72) Erfinder: Miftari, Filiz, 46282 Dorsten (DE)
(74) Vertreter: Von Rohr Patentanwälte Partnerschaft mbB

(56) Entgegenhaltungen:
- DE-A1- 10 238 353
- DE-A1-102007 021 482
- DE-A1-102010 002 863
- DATABASE GNPD [Online] MINTEL; 15. Februar 2018 (2018-02-15), anonymous: "Ultimate Hair Oil", XP055522445, gefunden im www.gnpd.com Database accession no. 5446661
- DATABASE GNPD [Online] MINTEL; 26. Januar 2018 (2018-01-26), anonymous: "Leave-in Conditioner", XP055522595, gefunden im www.gnpd.com Database accession no. 5398845

## Beschreibung

Die vorliegende Erfindung betrifft das technische Gebiet der Kosmetik, insbesondere das Gebiet der Haarpflege und Behandlung von Haaren.

Insbesondere betrifft die vorliegende Erfindung eine Zusammensetzung, bevorzugt ein Haaröl, und darüber hinaus die Verwendung der Zusammensetzung bzw. des Haaröls für kosmetische Zwecke, insbesondere zur Behandlung keratinischer Fasern.

Bei Haaren handelt es sich um lange, fadenförmige Horngebilde, welche vorwiegend aus dem Protein Keratin gebildet sind. Das einzelne Haar weist dabei drei Schichten auf, nämlich die äußere Cuticula, den Cortex und die Medulla.

Die Cuticula wird synonym auch als Schuppenschicht bezeichnet und besteht aus flachen, übereinander greifenden, verhornten, abgestorbenen Zellen. Bei gut gepflegten bzw. gesunden Haaren liegt die Schuppenschicht flach an und bildet eine glatte, durchscheinende Oberfläche aus, so dass das Licht reflektiert wird und das Haar glänzt. Der Cortex (Rinde) wird synonym auch als Faserschicht bzw. Faserstamm bezeichnet und macht mit einem Anteil von circa 80 % den größten Teil des Haares aus. Der Cortex wird durch Faserbündel, welche auf einer Vielzahl von Keratinfasern bzw. Fibrillen basieren, gebildet. Unter der Medulla (Mark des Haares) wird der innenliegende Kern des Haares verstanden, welcher ebenfalls aus Keratin besteht. Die Haare sind zudem in ihrem unteren Bereich über die Haarwurzel und den Haarfollikel in der Lederhaut verankert.

Haare, insbesondere die Kopfhaare des Menschen, unterliegen einem stetigen Wachstumszyklus, d.h. nach einer Phase des Wachstums fällt am Ende eines Zyklus das Haar aus und im verbleibenden Haarfollikel bildet sich ein neues Haar. Das menschliche Kopfhaar hat in der Regel eine Wachstumsphase von ca. 2 bis 6 Jahren, bevor es ausfällt und ersetzt wird.

Vor dem Hintergrund, dass die Talgdrüsen in der Kopfhaut konstant Talg produzieren, welcher wiederum Rückstände im Haar hinterlässt und die Haare schnell fettig aussehen lässt, und die Haare zudem im Alltag verschiedensten Belastungen ausgesetzt sind, stellt die Haarpflege üblicherweise einen wichtigen Bestandteil der Körperpflege beim Menschen dar. Auch kommt hinzu, dass sich ein einmal geschädigtes Haar biologisch nicht selbst regenerieren bzw. reparieren kann und Schäden an der Haarstruktur nur durch entsprechende Pflege reduziert oder verhindert werden können.

Das am häufigsten auftretende Problem bei Haaren ist eine übermäßige Austrocknung. Bereits gewöhnliche und unvermeidbare Umwelteinflüsse, wie UV-Strahlung, Chlor- oder Salzwasser und die regelmäßige Haarwäsche, fördern ein Austrocknen der Haare. Dies gilt insbesondere für lange Haare, da die Haarlängen bzw. Haarspitzen nicht mehr von dem natürlichen Rückfettungsprozess der Kopfhaut, welcher von den Talgdrüsen ausgeht, erreicht werden. Das Resultat sind ausgetrocknete Haarspitzen und Haarlängen, was wiederum Spliss, d.h. eine Spaltung der Haarspitzen, und Haarbruch begünstigt.

Weiterhin führen auch mechanische Beanspruchungen, z.B. durch äußere Reibung, insbesondere an der Kleidung, sowie ein häufiges Bürsten zu einer Schädigung der Haare. In Folge der mechanischen Beanspruchung wird die äußere Schicht der einzelnen Haare, d.h. die Cuticula, angeraut, was das Haar strohig, stumpf und kraus erscheinen lässt.

Vor allem das Haar von Frauen wird darüber hinaus durch den Einsatz von austrocknenden Stylingprodukten, wie Schaumfestiger, Haarspray oder Haargel, in Mitleidenschaft gezogen. Insbesondere Colorationen oder Haartönungen, welche vorzugsweise unter dem Einsatz von Oxidationsmitteln zur Öffnung der Haarstruktur für eine bessere Aufnahme der Pigmente durchgeführt werden, führen oftmals zu einer nachhaltigen Schädigung der Haarstruktur. Zudem trocknet auch der Einsatz von mit Hitze arbeitenden Stylinggeräten, wie Glätteisen, Lockenstäben oder Haartrocknern, das Haar oftmals so stark aus, dass es zu Schäden an der Haarstruktur kommt.

Eine Schädigung der Haarstruktur wiederum führt zu glanz- und kraftlosem Haar sowie Spliss und Haarbruch. Häufig bildet sich vor allem bei Haarbruch durch die verbliebenen kürzeren Haare bzw. die langsam nachwachsenden Haare ein sogenannter "Frizz" aus, welcher das Haar strohig, zerzaust und insgesamt ungepflegt erscheinen lässt. Darüber hinaus ist geschädigtes und trockenes Haar schwer zu bändigen und lässt sich nur schlecht kämmen, stylen bzw. in Form bringen. Liegt ein schwerwiegender Nährstoffmangel im Bereich der Haarwurzel bzw. Haarfollikel vor, kann dieser sogar zu einem übermäßigen Haarausfall führen.

Es existiert am Markt zwar eine Vielzahl von Produkten, um die vorgenannten Probleme zu lindern, zum Beispiel DE 10 2007 021482 A1 und DE 102 38 353 A1, allerdings werden mit den aus dem Stand der Technik bekannten Produkten oftmals nicht die gewünschten Wirkungen, teilweise sogar gegenteilige Effekte erzielt. Darüber hinaus enthalten viele Haarpflegeprodukte gesundheits- und umweltschädliche Stoffe, wie nachfolgend ausgeführt.

So beschreibt die DE 10 2009 028 537 A1 ein kosmetisches Mittel auf Basis von Öl oder Fett, welches zur kosmetischen Behandlung der Haare eingesetzt wird. Neben Ölen bzw. Fetten enthalten die Zusammensetzungen auch Silikone, insbesondere Dimethicone, Amodimethicone und Dimethiconole, um die Haare zu glätten und die Kämmbarkeit zu verbessern. Der Einsatz von Silikonen bzw. Silikonölen in Haarpflegeprodukten führt jedoch zu der Ausbildung eines durchgehenden Silikonfilms auf dem Haar ("Build-up-Effekt"), was wiederum zu einem unerwünschten Beschweren der Haare führt. Die Haare wirken dann oft fettig und hängen kraftlos und ohne Volumen herunter.

Weiterhin beschreibt die WO 2012/084339 A2 ein Haarbehandlungsmittel, welches zur Verbesserung des Glanzes, des Feuchtehaushalts, zum Schutz vor oxidativen Schädigungen sowie zum Reduzieren des Nachfettens eingesetzt wird und auf Polyurethan in Kombination mit mindestens einer quartären Ammoniumverbindung basiert. Bei quartären Ammoniumverbindungen handelt es sich um Filmbildner, welche auf den Haaren ebenfalls zum "Build-up-Effekt" führen und dieses in unerwünschter Weise beschweren.

Zahlreiche Haarpflegezusammensetzungen enthalten darüber hinaus verschiedenste umweltschädigende bzw. gesundheitsgefährdende Inhaltsstoffe. So werden häufig Parabene, insbesondere Methylparaben, Ethylparaben, Propylparaben und Isopropylparaben, als Konservierungsmittel eingesetzt. Für zahlreiche Parabene wird jedoch vermutet, dass diese eine dem weiblichen Geschlechtshormon Östrogen ähnelnde hormonelle Wirksamkeit aufweisen. Im Falle einer Einlagerung in den Körper wird davon ausgegangen, dass Parabene hormonbedingte Phänomene und Krankheiten, wie z.B. eine verfrühte Pubertät, Unfruchtbarkeit und verschiedene Krebserkrankungen, begünstigen können.

Gleichermaßen werden in Haarpflegeprodukten häufig Phthalate als Weichmacher eingesetzt, welche jedoch aufgrund ihrer Fett- und Wasserlöslichkeit über die Haut aufgenommen werden und so gegebenenfalls ins Blut gelangen. Bei Phthalaten wird ebenfalls von einer gewissen hormonellen Wirksamkeit ausgegangen, insbesondere einer Wirkung auf die Bauchspeicheldrüse.

Die vorgenannten Inhaltsstoffe sind zudem biologisch nicht abbaubar und gelangen über das Abwasser in den Wasserkreislauf. Im Ergebnis stellen zahlreiche in der Kosmetikindustrie verwendete Inhaltsstoffe sowohl für den Anwender als auch für die Umwelt eine nicht unerhebliche Belastung bzw. Gefahr dar.

Vor diesem Hintergrund sind im Stand der Technik bereits Ansätze getätigt worden, Kosmetikprodukte und Haarpflegeprodukte auf natürlicher Basis in Form von sogenannter Naturkosmetik bereitzustellen. Die diesbezüglich hergestellten Haarpflegeprodukte zeichnen sich zwar grundsätzlich gegenüber synthetischen bzw. konventionellen Zusammensetzungen oder Pflegeprodukten durch eine verbesserte Verträglichkeit für Mensch und Umwelt aus, führen aber oftmals nicht zu einem zufriedenstellenden Ergebnis im Hinblick auf die Haarpflege, insbesondere die Verbesserung der Haarstruktur.

Somit besteht weiterhin ein Bedarf an kosmetischen Zusammensetzungen zur Pflege bzw. Behandlung der Haare, welche einerseits eine gute Wirksamkeit bei der Verbesserung der Haarstruktur gewährleisten, insbesondere im Hinblick auf Glanz, Halt, Fülle, Kämmbarkeit und eine Behebung von Schäden, neben den pflegenden Eigenschaften jedoch auch eine verbesserte Verträglichkeit und biologische Abbaubarkeit besitzen.

Der vorliegenden Erfindung liegt daher die Aufgabe zugrunde, eine Zusammensetzung bereitzustellen, welche die zuvor beschriebenen Nachteile vermeidet oder aber wenigstens abschwächt.

Insbesondere liegt der vorliegenden Erfindung die Aufgabe zugrunde, eine Zusammensetzung bereitzustellen, welche eine gute Wirksamkeit bei der Behandlung von geschädigtem Haar bzw. bei der Vorbeugung von Schäden an der Haarstruktur, insbesondere in Bezug auf menschliches Kopfhaar, besitzt, und gleichzeitig eine gute (Umwelt-)Verträglichkeit und biologische Abbaubarkeit aufweist.

Zur Lösung der zuvor geschilderten Aufgabe schlägt die vorliegende Erfindung eine Zusammensetzung, insbesondere ein Haaröl, gemäß Anspruch 1 vor; weitere, insbesondere vorteilhafte Ausgestaltungen sind Gegenstand der diesbezüglichen abhängigen Ansprüche.

Ein weiterer Gegenstand der vorliegenden Erfindung ist zudem die Verwendung einer kosmetischen Zusammensetzung gemäß dem diesbezüglichen unabhängigen Anspruch.

Es versteht sich von selbst, dass im Folgenden besondere Ausgestaltungen, Ausführungsformen oder dergleichen, welche nur im Zusammenhang mit einem Erfindungsaspekt beschrieben sind, auch in Bezug auf die übrigen Erfindungsaspekte entsprechend gelten, ohne dass dies einer ausdrücklichen Erwähnung bedarf.

Weiterhin ist bei allen nachstehend genannten relativen bzw. prozentualen, insbesondere gewichtsbezogenen oder volumenbezogenen Mengenangaben zu beachten, dass diese im Rahmen der vorliegenden Erfindung vom Fachmann derart auszuwählen sind, dass in der Summe der jeweiligen Inhaltsstoffe, Wirkstoffe, Zusatz- bzw. Hilfsstoffe oder dergleichen stets 100 % bzw. 100 Gew.-% oder 100 Vol.-% resultieren. Dies versteht sich für den Fachmann aber von selbst.

Im Übrigen gilt, dass der Fachmann anwendungsbezogen oder einzelfallbedingt von den nachfolgend aufgeführten Zahlen-, Bereichs- oder Mengenangaben abweichen kann, ohne dass er den Rahmen der vorliegenden Erfindung dabei verlässt.

Zudem gilt, dass alle im Folgenden genannten Parameterangaben oder dergleichen grundsätzlich mit genormten oder explizit angegebenen Bestimmungsverfahren oder aber mit dem Fachmann an sich geläufigen Bestimmungsmethoden bestimmt bzw. ermittelt werden können.

Dies vorausgeschickt, wird nun die vorliegende Erfindung nachfolgend im Detail erläutert.

Gegenstand der vorliegenden Erfindung - gemäß einem **ersten** erfindungsgemäßen Aspekt - ist somit eine kosmetische Zusammensetzung, insbesondere in Form eines Haaröls, wobei die Zusammensetzung als Inhaltsstoffe Kokosöl, Olivenöl, Sojaöl, Mandelöl, Walnussöl und Jojobaöl gemäß Anspruch 1 enthält.

Im Rahmen der vorliegenden Erfindung wurde vollkommen überraschend gefunden, dass ein Haaröl auf Basis der zielgerichtet ausgewählten Kombination von natürlichen Ölen aus Kokos, Olive, Soja, Walnuss, Mandel und Jojoba eine hervorragende Wirkung bei der Behandlung keratinischer Fasern, insbesondere des menschlichen Kopfhaars, besitzt. Insbesondere ist die Zusammensetzung sowohl als Schutz vor Schäden an der Haarstruktur als auch zur Linderung oder Beseitigung bereits vorhandener Schäden geeignet. Gleichzeitig treten keine unerwünschten oder nachteiligen Effekte auf. Insbesondere der oftmals bei der Verwendung von Zusammensetzungen mit Silikonen oder quartären Ammoniumverbindungen auftretende "Build-up-Effekt" kann vermieden werden. Zudem basiert das Haaröl zumindest im Wesentlichen auf natürlichen Inhaltsstoffen, so dass eine gute Verträglichkeit, Umweltfreundlichkeit bzw. biologische Abbaubarkeit der Zusammensetzung gewährleistet ist.

Die erfindungsgemäße Zusammensetzung bzw. das Haaröl nach der vorliegenden Erfindung besitzt eine Vielzahl von Vorteilen und Besonderheiten, welche nachfolgend geschildert sind und als Indiz für das Vorliegen einer erfinderischen Tätigkeit zu werten sind:
Die Zusammensetzung nach der vorliegenden Erfindung wirkt feuchtigkeitsspendend auf das behandelte Haar. Dabei besitzt das Haaröl eine hervorragende Tiefenwirkung und dringt tief in sprödes und strapaziertes Haar ein. Die äußere Schuppenschicht der Haare wird auf dieser Basis geglättet, das Haar erhält einen natürlichen Glanz und kraftlos wirkendes Haar wirkt wieder füllig. Dabei wird auch die Kämmbarkeit der Haare, insbesondere nach der Haarwäsche, verbessert. Darüber hinaus führt die Erhöhung der Feuchtigkeit in den Haaren, insbesondere den Haarspitzen, zu einer Reduktion von Haarbruch und Spliss. Durch Haarbruch und Spliss bereits kraus wirkendes Haar bzw. sogenanntes "Frizz-Hair" wird ebenfalls geglättet und wird wieder geschmeidig.

Weiterhin lässt sich mit dem erfindungsgemäßen Haaröl bei bereits geschädigtem Haar ein hervorragender "Repair"-Effekt erzielen, auf dessen Basis - ohne sich dabei auf diese Theorie beschränken zu wollen - die aufgeraute und schuppige äußere Haarschicht, d.h. die Cuticula oder Schuppenschicht, geglättet wird und das einzelne Haar wieder eine glatte, durchscheinende Oberfläche erhält. Durch eine Glättung der Cuticula wird das Haar wieder geschmeidig und kann das Licht optimal reflektieren, was dem Haar einen natürlichen Glanz verleiht.

Zudem schützt das erfindungsgemäße Haaröl die Haare in hervorragender Weise vor hitzebedingten Schäden, welche vor allem bei der Benutzung von Hitze entwickelnden Stylinggeräten, insbesondere Haartrocknern, Lockenstäben oder Glätteisen, auftreten können.

Weiterhin zeichnet sich die erfindungsgemäße Zusammensetzung durch ihre hervorragende Verträglichkeit aus, da sie zumindest im Wesentlichen nur natürliche Inhaltsstoffe enthält, nämlich pflanzliche Öle und gegebenenfalls auf natürlichen Inhaltsstoffen basierende Emulgatoren und Konservierungsmittel.

Das Haaröl gemäß der vorliegenden Erfindung ist auf Basis der natürlichen Inhaltsstoffe reich an wertvollen Mineralien und ungesättigten Fettsäuren, so dass die Haare mit den erforderlichen Vitaminen und Inhaltsstoffen versorgt werden, welche es kräftiger und gesund erscheinen lassen. Aufgrund der Reichhaltigkeit des Öls - ohne sich hierbei auf diese Theorie beschränken zu wollen - kann das Haaröl sogar zur Linderung von Haarausfall, welcher durch einen Nährstoffmangel im Bereich der Haarwurzel und Haarfollikel bedingt ist, gelindert oder reduziert werden.

Darüber hinaus ist die Zusammensetzung frei von den oftmals in Haarpflegeprodukten enthaltenen Silikonen bzw. Silikonölen, quartären Ammoniumverbindungen, Parabenen, Sulfaten und Phthalaten. Die vorgenannten Inhaltsstoffe führen nämlich oftmals zu unerwünschten Effekten bei der Behandlung der Haare, insbesondere dem unerwünschten "Build-up-Effekt". Gleichermaßen werden die für die vorgenannten Inhaltsstoffe vermuteten negativen Effekte und Wirkungen auf Mensch und Umwelt, wie die Erzeugung von Hautreizungen, eine hormonähnliche Wirkung und schlechte biologische Abbaubarkeit, vermieden.

Zur Vermeidung weiterer Nebenwirkungen und Hautreizungen enthält das Haaröl zudem vorzugsweise auch kein zugesetztes Parfüm, da Parfüm- und Aromastoffe ebenfalls zu unerwünschten Hautreizungen führen können. Insbesondere kann auf dieser Basis auch das Risiko von allergischen Reaktionen gesenkt werden.

Darüber hinaus ist das Haaröl gemäß der vorliegenden Erfindung äußerst komfortabel in der Handhabung. Denn die zielgerichtete Kombination der verschiedenen Öle, insbesondere wenn zusätzlich ein Emulgator verwendet wird, führt zu einer Konsistenz bzw. Viskosität oder Dünnflüssigkeit, welche unterhalb der Viskosität von gewöhnlichen Ölen, wie z.B. reinem Kokosöl oder Olivenöl, liegt. Auf dieser Basis kann das Öl besonders fein und gleichmäßig im Haar verteilt werden, so dass keine fettig wirkenden Rückstände in den Haaren verbleiben.

Schließlich ist die Zusammensetzung nach der vorliegenden Erfindung auch äußerst flexibel und vielseitig im Bereich der Haarpflege verwendbar:
So kann die erfindungsgemäße Zusammensetzung als Haarpflege nach der Haarwäsche aufgetragen und in das feuchte Haar einmassiert werden. Ein Auswaschen des Haaröls ist trotz des hohen Fettanteils nicht notwendig. Nach Anwendung der Zusammensetzung lässt sich das Haar deutlich besser kämmen und stylen, da die Oberfläche der Haare geglättet wird und die statische Aufladung der Haare reduziert wird. Darüber hinaus sehen die Haare gesünder aus, sind geschmeidiger und der natürliche Glanz wird unterstützt.

Weiterhin kann die erfindungsgemäße Zusammensetzung auch als intensive Haarkur bzw. Haarmaske verwendet werden. Dazu wird das Öl möglichst gleichmäßig verteilt, in die Haare einmassiert und nach einer mehrstündigen Einwirkzeit ausgespült. Auf dieser Basis wird eine noch intensivere Pflegewirkung erzielt, da die Nährstoffe und Mineralien bis in die Haarfollikel bzw. Haarwurzeln vordringen können. Durch Neutralisierung des pH-Werts auf der Kopfhaut wird ein schnelles Nachfetten verhindert und gleichzeitig der Feuchtigkeitshaushalt der Kopfhaut reguliert, d.h. einem Austrocknen entgegengewirkt. Zudem bietet die Anwendung des Öls als Maske einen Schutz vor Haarausfall, welcher durch Nährstoffmangel bedingt ist.

Weiterhin kann das erfindungsgemäße Haaröl als Styling-Finish und Hitzeschutz beim Styling verwendet werden. Dabei verleiht es den Haaren einen intensiven Glanz, Lebendigkeit und verhindert ein Austrocknen der Haarspitzen und hitzebedingte Schäden.

Schließlich kann das Haaröl auch Haarfarbe oder Haartönungen beim privaten Colorieren der Haare oder im Friseursalon zugesetzt werden. Dabei wird je nach Haardichte und Haarstruktur die erforderliche Menge des Haaröls in die Haarfarbe gemischt. Auf dieser Basis wird das beim Färben oder Tönen für Austrocknung, Haarbruch und Spliss besonders anfällige Haar geschützt. Die Haare sind nach dem Färben weicher und geschmeidiger und eine leichte Kämmbarkeit wird gewährleistet. Insbesondere kann auch der insbesondere beim Kämmen von frisch gefärbten Haaren oftmals auftretende Haarverlust signifikant reduziert werden. Insgesamt können mit der erfindungsgemäßen Zusammensetzung die beim Colorieren, insbesondere Blondieren, häufig auftretenden unerwünschten Begleiterscheinungen verhindert bzw. minimiert werden.

Die vorgenannten Vorteile und Besonderheiten sind als Indiz für das Vorliegen einer erfinderischen Tätigkeit zu werten. Nachfolgend werden zum besseren Verständnis zudem Definition und Erklärungen zu den im Rahmen der vorliegenden Erfindung verwendeten Begrifflichkeiten gegeben.

Im Rahmen der vorliegenden Erfindung wird unter dem Begriff "Kokosöl" (synonym auch als *Oleum cocos* Kokosnussöl oder Kokosfett bezeichnet) ein Pflanzenfett verstanden, welches aus dem Nährgewebe der Frucht der Kokospalme, d.h. der Kokosnuss, aus der Kokospalme (*Cocos nucifera*) gewonnen wird. Kokosöl enthält als Inhaltsstoffe insbesondere Ölsäure, Linolsäure, Linolensäure, Palmitinsäure, Laurinsäure, Myristinsäure, eine Reihe weiterer Fettsäuren sowie Vitamin E bzw. Tocopherol. Kokosöl besitzt feuchtigkeitsspendende Effekte und wirkt pflegend bzw. feuchtigkeitsregulierend auf Haare, Haarwurzeln und Kopfhaut.

Unter dem Begriff "Olivenöl" wird erfindungsgemäß ein Pflanzenöl verstanden, welches aus dem Fruchtfleisch und dem Kern von Früchten des Olivenbaums (*Olea europaea*) gewonnen wird. Olivenöl enthält als Inhaltsstoffe in erster Linie Ölsäure, Linolsäure und Palmitinsäure sowie Vitamin A und Chlorophylle und Carotinoide als sekundäre Pflanzenstoffe. Olivenöl wirkt auf Haare ebenfalls feuchtigkeitsspendend und schützt vor übermäßiger Hitze. Gleichermaßen hilft Olivenöl gegen sprödes und splissiges Haar und sorgt für den allgemein erwünschten Glanz, ohne jedoch fettig zu wirken.

Was darüber hinaus das im Rahmen der vorliegenden Erfindung eingesetzte Mandelöl anbelangt, so wird hierunter im Rahmen der vorliegenden Erfindung insbesondere das aus süßen Mandeln, d.h. den Früchten von *Prunus amygdalus dulcis,* gewonnene Pflanzenöl verstanden. Mandelöl besteht vorwiegend aus den Fettsäuren Ölsäure, Linolsäure, Palmitinsäure und Stearinsäure. Darüber hinaus kann Mandelöl Vitamine, insbesondere Vitamin E bzw. Tocopherole, sowie Biotin enthalten, welche wichtige Nährstoffe für die Haare darstellen.

Unter dem Begriff "Jojobaöl" (synonym auch Jojobawachs) wird im Rahmen der vorliegenden Erfindung zudem das aus den Samen des Jojobastrauchs (*Simmondsia chinensis*) gewonnene Öl bzw. Wachs verstanden. Jojobaöl bzw. Jojobawachs weist als Inhaltsstoffe insbesondere Gandoleinsäure, Erucasäure und Ölsäure auf. Darüber hinaus ist Jojobaöl reich an Vitaminen, insbesondere Provitamin A, Vitamin E und Vitamin B. Darüber hinaus wirkt es Haarbruch, Spliss und "Frizz" entgegen, so dass mit dem Einsatz von Jojobaöl in den erfindungsgemäßen Zusammensetzungen auch ein "Repair-Effekt" einhergeht. Weiterhin wirkt Jojobaöl antibakteriell und besitzt einen pflegenden Effekt auf Haare und Kopfhaut.

Unter dem Begriff "Walnussöl", wie er im Rahmen der vorliegenden Erfindung verwendet wird, wird das aus den Samen von Walnüssen (*Juglans* Sp.) gewonnenes Öl verstanden. Walnüssöl enthält als Inhaltsstoffe in erster Linie Ölsäure, Linolsäure, Linolensäure, Palmitinsäure und Stearinsäure als Fettsäuren und darüber hinaus Vitamine, insbesondere Tocopherol bzw. Vitamin E.

Unter dem Begriff "Sojaöl" wird im Rahmen der vorliegenden Erfindung schließlich ein aus der Sojabohne (*Glycine max*) gewonnenes Pflanzenöl verstanden, welches als Fettsäuren in erster Linie Ölsäure, Linolsäure, Linolensäure und Palmitinsäure enthält und darüber hinaus reich an Vitaminen, insbesondere Tocopherol bzw. Vitamin E, ist.

Im Rahmen der vorliegenden Erfindung hat sich vollkommen überraschend gezeigt, dass gerade die spezielle Kombination von Kokosöl, Olivenöl, Mandelöl, Sojaöl, Jojobaöl und Walnussöl gemäß Anspruch 1 die Bereitstellung eines Haaröls mit gegenüber bekannten Zusammensetzungen deutlich verbesserter Wirksamkeit im Hinblick auf Verbesserung der Haarstruktur, Glanz, Kämmbarkeit, Geschmeidigkeit, Reduzierung von elektrostatischer Aufladung und verhindern eines "Build-up-Effekts" gewährleistet. Insbesondere war in diesem Zusammenhang überraschend, dass eine maßgeblich auf Ölen basierende Zusammensetzung nach der Anwendung im Haar verbleiben kann, ohne dass das Haar fettig wirkt oder beschwert wird.

Insbesondere geht die Wirkung der jeweils einzelnen Öle bei Verwendung in der zielgerichteten Kombination mit den übrigen Ölen über die Wirkung bei alleiniger Verwendung hinaus, so dass in Bezug auf die spezielle Kombination an Inhaltsstoffen von Ölen von einem synergistischen Wirkeffekt bei der Behandlung keratinischer Fasern auszugehen ist.

Die erfindungsgemäße Zusammensetzung kann in vielfältiger Weise ausgestaltet sein. Bevorzugte Ausführungsformen der erfindungsgemäßen Zusammensetzung sind nachfolgend im Detail beschrieben:
Neben der Auswahl bzw. Kombination der erfindungsgemäß eingesetzten Öle als solcher hat sich zudem deren jeweils eingesetzte Menge als kritisch im Hinblick auf die Wirksamkeit erwiesen.

Was die Menge des Kokosöls anbelangt, so kann diese in weiten Bereichen variieren. Eine besonders gute Wirksamkeit der erfindungsgemäßen Zusammensetzung wird erzielt, wenn die Zusammensetzung das Kokosöl in einer Menge im Bereich von 20 bis 70 Vol.-%, vorzugsweise 30 bis 50 Vol.-%, bevorzugt 35 bis 45 Vol.-%, bezogen auf die Zusammensetzung, enthält.

Gemäß einer bevorzugten Ausführungsform der vorliegenden Erfindung kann es zudem vorgesehen sein, dass die Zusammensetzung das Olivenöl in einer Menge im Bereich von 1 bis 20 Vol.-%, bevorzugt 5 bis 15 Vol.-%, besonders bevorzugt 8 bis 12 Vol.-%, bezogen auf die Zusammensetzung, enthält.

Im Hinblick auf das Sojaöl ist es zudem vorteilhaft, wenn die Zusammensetzung das Sojaöl in einer Menge im Bereich von 1 bis 20 Vol.-%, bevorzugt 5 bis 15 Vol.-%, besonders bevorzugt 8 bis 12 Vol.-%, bezogen auf die Zusammensetzung, enthält.

Zudem kann es erfindungsgemäß vorgesehen sein, dass die Zusammensetzung das Mandelöl in einer Menge im Bereich von 1 bis 20 Vol.-%, bevorzugt 5 bis 15 Vol.-%, besonders bevorzugt 8 bis 12 Vol.-%, bezogen auf die Zusammensetzung, enthält.

Weiterhin ist es vorteilhaft, wenn die Zusammensetzung das Walnussöl in einer Menge im Bereich von 1 bis 20 Vol.-%, bevorzugt 5 bis 15 Vol.-%, besonders bevorzugt 8 bis 12 Vol.-%, bezogen auf die Zusammensetzung, enthält.

Im Hinblick auf das Jojobaöl hat es sich schließlich ebenfalls als vorteilhaft erwiesen, wenn die Zusammensetzung das Jojobaöl in einer Menge im Bereich von 1 bis 20 Vol.-%, bevorzugt 5 bis 15 Vol.-%, besonders bevorzugt 8 bis 12 Vol.-%, bezogen auf die Zusammensetzung, enthält.

Im Rahmen der vorliegenden Erfindung hat sich zudem überraschend gezeigt, dass neben den Mengen der einzelnen Öle auch deren volumenbezogenes Verhältnis zueinander einen Einfluss auf die Eigenschaften, insbesondere die Wirksamkeit einerseits und die rheologischen Eigenschaften andererseits, nimmt. Insbesondere ist es möglich, über die Mengen- bzw. Volumenverhältnisse zueinander die physiko-chemischen Eigenschaften der Zusammensetzungen derart zu steuern, dass die Zusammensetzung gegenüber herkömmlichen Ölen eine herabgesetzte Viskosität besitzt und sich auf dieser Basis besonders gut und gleichmäßig im Haar verteilen lässt, ohne dass dabei beschwerende Rückstände verbleiben oder die Haare fettig wirken würden.

In diesem Zusammenhang hat es sich als besonders vorteilhaft erwiesen, wenn die Zusammensetzung Olivenöl, Sojaöl, Mandelöl, Walnussöl und Jojobaöl jeweils zu zumindest im Wesentlichen gleichen Volumenanteilen enthält. Mit anderen Worten ist es gemäß einer bevorzugten Ausführungsform der vorliegenden Erfindung vorgesehen, dass die Zusammensetzung Olivenöl, Sojaöl, Mandelöl, Walnussöl und Jojobaöl in einem volumenbezogenen Verhältnis von Olivenöl : Sojaöl : Mandelöl : Walnussöl : Jojobaöl von zumindest im Wesentlichen 1 : 1 : 1 : 1 : 1 enthält.

Gleichermaßen hat es sich in diesem Zusammenhang als vorteilhaft erwiesen, wenn die Zusammensetzung das Kokosöl und die weiteren Öle [Olivenöl, Sojaöl, Mandelöl, Walnussöl und Jojobaöl] in einem volumenbezogenen Verhältnis von Kokosöl zu den weiteren Ölen [Olivenöl, Sojaöl, Mandelöl, Walnussöl und Jojobaöl] im Bereich von 1 : 10 bis 10 : 1, insbesondere 1 : 5 bis 5 : 1, vorzugsweise 1 : 3 bis 3 : 1, bevorzugt 1 : 2 bis 1 : 1, enthält. Das Mengenverhältnis von Kokosöl zu den übrigen Ölen bezieht sich dabei auf das eingesetzte Volumen an Kokosöl zu der Summe der jeweils eingesetzten Volumina der übrigen Öle.

Darüber hinaus können die physiko-chemischen Eigenschaften der erfindungsgemäßen Zusammensetzung, insbesondere die Homogenität und Stabilität der Zusammensetzung, verbessert werden, wenn die Zusammensetzung als weiteren Inhaltsstoff mindestens einen Emulgator enthält. Auf Basis des zusätzlichen Emulgators wird das Haaröl weiterführend stabilisiert und einer Phasentrennung oder dem Absetzen eines Inhaltsstoffs vorgebeugt.

Dabei kann die Zusammensetzung nach der vorliegenden Erfindung als Öl-in-Wasser-Emulsion oder als Wasser-in-ÖI-Emulsion, vorzugsweise als Öl-in-Wasser-Emulsion, ausgebildet sein.

Im Hinblick auf den Einsatz eines Emulgators hat es sich als besonders vorteilhaft erwiesen, wenn die Zusammensetzung den Emulgator in einer Menge im Bereich von 0,01 bis 10 Vol.-%, insbesondere 0,1 bis 8 Vol.-%, vorzugsweise 1 bis 5 Vol.-%, bezogen auf die Zusammensetzung, enthält.

Zur Erzielung einer besonders guten Emulgatorwirkung kann es vorgesehen sein, dass der Emulgator Phospholipide, insbesondere Phosphatidylcholine (Lecithine), enthält. Diesbezüglich hat es sich als vorteilhaft erwiesen, wenn der Emulgator die Phospholipide in einer Menge im Bereich von 20 bis 90 Vol.-%, vorzugsweise 30 bis 80 Vol.-%, bevorzugt 40 bis 70 Vol.-%, bezogen auf den Emulgator, enthält.

Darüber hinaus kann es auch vorgesehen sein, dass der Emulgator mindestens ein pflanzliches Öl, insbesondere Distelöl und/oder Sonnenblumenöl, vorzugsweise Distelöl, enthält. Sowohl Diestelöl als auch Sonnenblumenöl sind nicht nur im Hinblick auf die Emulgatorwirkung von Bedeutung, sondern wirken auch feuchtigkeitsspendend und pflegend auf Haare und Kopfhaut.

Was die Mengen des pflanzlichen Öls anbelangt, so können diese in weiten Bereichen variieren. Besonders gute Ergebnisse werden erhalten, wenn der Emulgator das mindestens eine pflanzliche Öl in einer Menge im Bereich von 20 bis 90 Vol.-%, vorzugweise 30 bis 80 Vol.-%, bevorzugt 40 bis 70 Vol.-%, bezogen auf den Emulgator, enthält.

Was den Emulgator weiterhin anbelangt, so kann es vorgesehen sein, dass dieser weitere kosmetische Hilfsstoffe und/oder Additive enthält, insbesondere ausgewählt aus der Gruppe von (i) Glycerin; (ii) mittelkettigen Triglyceriden (MCT-Fette), insbesondere Capronsäuren, Caprylsäuren, Caprinsäuren, Laurinsäuren sowie deren kosmetisch verträglichen Salzen, vorzugsweise Caprylsäuren und/oder Caprinsäuren sowie deren kosmetisch verträglichen Salzen; (iii) Alkoholen, insbesondere Ethanol; (iv) Glycinen und/oder deren kosmetisch verträglichen Salzen, insbesondere Glycerinstearinsäure und/oder deren kosmetisch verträgliche Salzen; (v) Palmitinsäuren und/oder deren kosmetisch verträglichen Salzen und/oder Estern, insbesondere Palmitinsäureascorbylester.

Geeignete Emulgatoren für erfindungsgemäße Zusammensetzungen sind beispielsweise unter den Produktbezeichnungen "Fluidlecithin Super", "Fluidlecithin Super HT" oder "Fluidlecithin CM" über die Spinnrad GmbH, Bad Segeberg, DE erhältlich.

Um die Wirksamkeit des erfindungsgemäßen Haaröls, insbesondere im Hinblick auf den "Repair-Effekt", weiterführend zu steigern, hat es sich zudem als vorteilhaft erwiesen, wenn die Zusammensetzung als weiteren Inhaltsstoff Proteine bzw. Hydrolysat von Proteinen enthält. Wie eingangs bereits ausgeführt, sind Haare in erster Linie auf Basis von Proteinen, insbesondere Keratin, ausgebildet. Die Proteinstruktur der Haare kann jedoch durch äußere Einflüsse, wie die Verwendung von Styling-Produkten, Sonnenstrahlen, Kälte, Styling-Geräte, insbesondere Glätteisen und Lockenstäbe, aber auch bereits durch unvermeidbare Umwelteinflüsse, z.B. UV-Strahlung, geschädigt werden, so dass das Haar an Glanz, Sprungkraft und Festigkeit verliert.

Durch den Zusatz von Proteinen in den erfindungsgemäßen ölhaltigen Zusammensetzungen können Schäden an der Haarstruktur reduziert werden, da sich die eingesetzten Proteine an die äußere Schuppenschicht der Haare anlagern und Strukturschäden oder "Löcher" in der Struktur auffüllen. Auf dieser Basis wird das Haar geglättet und insgesamt stabilisiert. Mit anderen Worten können Strukturschäden am Keratin der Haare - ohne sich hierbei auf diese Theorie beschränken zu wollen - durch in der erfindungsgemäßen Zusammensetzung enthaltene Proteine aufgefüllt und repariert werden.

In diesem Zusammenhang hat es sich als besonders vorteilhaft erwiesen, wenn die erfindungsgemäße Zusammensetzung die Proteine bzw. Hydrolysat von Proteinen in einer Menge im Bereich von 0,001 bis 5 Gew.-%, insbesondere 0,01 bis 4 Gew.-%, vorzugsweise 0,1 bis 3 Gew.-%, bezogen auf die Zusammensetzung, enthält.

Besonders gute Ergebnisse werden in diesem Zusammenhang erhalten, wenn die Proteine und/oder die Hydrolysate von Proteinen aus der Gruppe von Proteinen tierischen Ursprungs sowie Hydrolysaten davon ausgewählt sind. Insbesondere können als Proteine Keratin, Elastine, Kollagene und/oder Seidenproteine sowie Hydrolysaten davon in der Zusammensetzung enthalten sein.

Gemäß einer besonders bevorzugten Ausführungsform der vorliegenden Erfindung sind die Proteine und/oder die Hydrolysate von Proteinen aus der Gruppe von Proteinen pflanzlichen Ursprungs sowie Hydrolysaten davon ausgewählt. Insbesondere sind die Proteine ausgewählt aus Sojaproteinen, Weizenproteinen und/oder Haferproteinen sowie Hydrolysaten davon. Die beste Wirkung wird erzielt, wenn die Zusammensetzungen Weizenproteine bzw. Hydrolysate von Weizenproteinen enthalten.

Auf dieser Basis kann der "Repair-Effekt" der erfindungsgemäßen Zusammensetzungen bei sprödem, porösem oder kraftlosem Haar noch weiterführend verstärkt werden, so dass dem Haar noch mehr Elastizität, Glanz und Stabilität verliehen wird.

Eine für die erfindungsgemäße Zusammensetzung geeignete Proteinzusammensetzung ist beispielsweise unter der Produktbezeichnung "Nuratin P" über die Jean Pütz Produkte GmbH, Gelsenkirchen, DE oder die Spinnrad GmbH, Bad Segeberg, DE unter der Artikelnummer 002240321 kommerziell erhältlich.

Im Hinblick auf die Haltbarkeit der Zusammensetzung nach der vorliegenden Erfindung hat es sich zudem als vorteilhaft erwiesen, wenn die erfindungsgemäße Zusammensetzung mindestens ein Konservierungsmittel enthält. Im Hinblick auf Verträglichkeit und biologische Abbaubarkeit ist es bevorzugt, ein auf natürlichen Inhaltsstoffen basierendes Konservierungsmittel einzusetzen.

Die Menge des Konservierungsmittels in den Zusammensetzungen kann in weiten Bereichen variieren und zielgerichtet auf das jeweils eingesetzte Konservierungsmittel und die übrigen Inhaltsstoffe der Zusammensetzung abgestimmt werden. Im Allgemeinen werden gute Ergebnisse erhalten, wenn die Zusammensetzung bzw. das Haaröl das mindestens eine Konservierungsmittel in einer Menge im Bereich von 0,01 bis 10 Vol.-%, insbesondere 0,05 bis 8 Vol.-%, vorzugsweise 0,1 bis 5 Vol.-%, bezogen auf die Zusammensetzung, enthält.

Was die Auswahl eines speziellen Konservierungsmittels anbelangt, kann es gemäß einer besonders bevorzugten Ausführungsform im Rahmen der vorliegenden Erfindung vorgesehen sein, dass das Konservierungsmittel Antioxidantien enthält, insbesondere Vitamin C (Ascorbinsäure und/oder deren Salze), Vitamin E (Tocopherole und/oder Tocotrienole) und/oder Citronensäure und/oder deren Salze. Die vorgenannten Inhaltsstoffe wirken zudem nicht nur konservierend, sondern schützen auch die Haare selbst vor Oxidation.

Ein für die erfindungsgemäßen Zusammensetzungen geeignetes natürliches Konservierungsmittel ist beispielsweise unter der Produktbezeichnung "Antiranz" kommerziell erhältlich.

Darüber hinaus ist es im Rahmen der vorliegenden Erfindung vorgesehen, dass die Zusammensetzung gemäß der vorliegenden Erfindung zumindest im Wesentlichen auf natürlichen Inhaltsstoffen bzw. biologisch abbaubaren Inhaltsstoffen basiert.

Es ist im Rahmen der vorliegenden Erfindung vorgesehen, dass die Zusammensetzung frei ist von Polyorganosiloxanen bzw. Polysiloxanen. Polyorganosiloxane bzw. Polysiloxane, synonym auch als Silikone bzw. Silikonöle bezeichnet, sind üblicherweise zur Verbesserung der Kämmbarkeit und Geschmeidigkeit der Haare in Haarpflegeprodukten enthalten. Dabei kommen in erster Linie Amodimethicone, Polydimethylsiloxan (PDMS), Cetyl Dimethicone, Cytopentasiloxan, Simethicone und Trimethylsilylamodimethicone zum Einsatz. Silikone bzw. Silikonöle können jedoch hautreizend sein und somit die Verträglichkeit der Pflegeprodukte beeinträchtigen. Darüber hinaus führen Haarprodukte mit Silikonen bei regelmäßiger Anwendung zu einem sogenannten "Build-up-Effekt", da sich die Silikone dauerhaft an die Haarstruktur anlagern und eine zusammenhängende Schicht auf den Haaren ausbilden, welche wiederum die Haare beschwert und kraftlos und fettig wirken lässt.

Weiterhin ist es gemäß einer bevorzugten Ausführungsform ebenfalls vorgesehen, dass die erfindungsgemäße Zusammensetzung frei ist von Parabenen. Parabene, insbesondere Methylparaben, Ethylparaben, Propylparaben, Butylparaben und Isopropylparaben, werden im Bereich der Haarpflege und Kosmetik häufig als Konservierungsmittel eingesetzt. Bei Parabenen wird jedoch von einer gewissen hormonellen Wirksamkeit, welche der Wirksamkeit des weiblichen Geschlechtshormons Östrogen ähnelt, ausgegangen. Parabene können in das menschliche Gewebe eindringen und dort vermutlich hormonbedingte Phänomene und Krankheiten zumindest begünstigen.

Gleichermaßen ist es erfindungsgemäß, dass die Zusammensetzung nach der vorliegenden Erfindung frei ist von Phthalsäuren sowie deren Salzen bzw. Estern. Phthalate, wie z. B. Dimethylphthalat oder Dietyhlphthalat, werden in der Kosmetik häufig als Weichmacher eingesetzt. Da sie sowohl fett- als auch wasserlöslich sind, können sie über die Haut aufgenommen werden und gelangen so ins Blut. Auch bei Phthalaten wird von einer hormonellen Wirksamkeit, insbesondere in Bezug auf die Bauchspeicheldrüse, ausgegangen.

Darüber hinaus ist es im Rahmen der vorliegenden Erfindung auch vorgesehen, dass die Zusammensetzung frei ist von Quaternium bzw. Polyquaternium, insbesondere quartären Ammoniumverbindungen, wie Polyquaternium-7, Polyquaternium-10, Polyquaternium-30 und dergleichen. Quartäre Ammoniumverbindungen der vorgenannten Art werden häufig aufgrund ihrer kationischen Eigenschaften als Filmbildner eingesetzt und bilden auf keratinhaltigen Oberflächen, wie den Haaren, einen zusammenhängenden Film aus, so dass die elektrostatische Aufladung verringert und die Kämmbarkeit verbessert wird. Wie auch die zuvor beschriebenen Silikone führen quartäre Ammoniumverbindungen zu dem unerwünschten "Build-up-Effekt". Darüber hinaus enthalten einzelne quartäre Ammoniumverbindungen, insbesondere Polyquaternium-7, umweltschädliches Mikroplastik, welches über das Abwasser in den Wasserkreislauf gelangt.

Gleichermaßen ist es erfindungsgemäß, dass die Zusammensetzung nach der vorliegenden Erfindung keine Sulfate mit schaumbildender bzw. reinigender Wirkung enthält. Die in zahlreichen kosmetischen Zusammensetzungen enthaltenden Sulfate mit schäumender Reinigungswirkung, insbesondere Natriumlaurylsulfat, führen zu einem Austrocknen der Haarstruktur, was durch Hitze, Styling und Colorationen geschädigtes bzw. stark beanspruchtes Haar weiter schädigen kann.

Schließlich ist es gemäß der Ausführungsform auch vorgesehen, dass die Zusammensetzung frei ist von zusätzlichen, insbesondere synthetischen Parfüm- oder Duftstoffen. Duftstoffe bzw. Parfumstoffe können ebenfalls zu Hautreizungen führen und erhöhen das Risiko allergischer Reaktionen.

Wie zuvor ausgeführt, ist es erfindungsgemäß, dass die erfindungsgemäße Zusammensetzung frei ist von den zuvor genannten, üblicherweise in Haarprodukten befindlichen Inhaltsstoffen. In diesem Zusammenhang war es vollkommen überraschend, dass auch ohne diese in der Kosmetikindustrie weit verbreiteten und im Hinblick auf die Wirkung anerkannten Wirkstoffe bzw. Inhaltsstoffe ausschließlich auf Basis von natürlichen Inhaltsstoffen, insbesondere Kokosöl, Jojobaöl, Olivenöl, Mandelöl, Sojaöl und Walnussöl, eine Zusammensetzung bzw. ein Haaröl zur Behandlung keratinischer Fasern, insbesondere des menschlichen Kopfhaars, bereitgestellt werden kann, welche bzw. welches sprödem, strapaziertem, brüchigem und trockenem Haar Glanz und Geschmeidigkeit, Feuchtigkeit und eine gute Kämmbarkeit ohne Haarverlust und ohne elektrostatische Aufladung verleiht. Im Gegensatz zu Produkten auf Basis der zuvor genannten Silikone, Parabene, quartären Ammoniumverbindungen, Sulfaten und Phthalaten ist das erfindungsgemäße Haaröl auch für eine längerfristige bzw. häufige Anwendung geeignet, da das Problem des Beschwerens bzw. des unerwünschten "Build-up-Effekts" vermieden werden kann, ohne jedoch an Wirksamkeit einzubüßen.

Zudem kann es erfindungsgemäß vorgesehen sein, dass die Zusammensetzung mindestens einen weiteren kosmetisch verträglichen Hilfsstoff und/oder mindestens ein weiteres kosmetisch verträgliches Additiv enthält. Ein solcher Hilfsstoff oder ein solches Additiv ist bevorzugt aus der Gruppe von (i) Stabilisatoren; (ii) Filmbildnern; (iii) Rheologiemodifikatoren; (iv) Duftstoffen und/oder Parfumstoffen; und/oder (v) Farbstoffen und/oder Pigmenten ausgewählt.

Im Ergebnis wird mit der vorliegenden Erfindung ein vielseitig einsetzbares Haaröl bzw. eine vielseitig einsetzbare Zusammensetzung bereitgestellt, welches bzw. welche einerseits auf natürlichen Inhaltsstoffe basiert und somit eine hervorragende Verträglichkeit und biologische Abbaubarkeit gewährleistet, dabei jedoch gleichzeitig vollkommen überraschend auch eine hervorragende Wirksamkeit bei der Behandlung von und dem Schutz vor sprödem, strapaziertem, brüchigem und trockenem Haar gewährleistet.

Darüber hinaus betrifft die vorliegende Erfindung - gemäß einem **zweiten** Aspekt der vorliegenden Erfindung - die Verwendung einer Zusammensetzung, wie sie zuvor beschrieben wurde, insbesondere des Haaröls nach der vorliegenden Erfindung, im Bereich der Kosmetik, insbesondere zur Behandlung und/oder Pflege keratinischer Fasern, vorzugsweise menschlicher Haare.

Wie zuvor bereits geschildert, zeichnet die erfindungsgemäße Zusammensetzung, insbesondere das Haaröl, durch eine breite Anwendbarkeit im Bereich der Kosmetik, insbesondere der Haarpflege, aus.

Gemäß einer bevorzugten Verwendung der erfindungsgemäßen Zusammensetzung kann es vorgesehen sein, diese als Haarpflege nach dem Duschen in das feuchte Haar einzumassieren und anschließend wie gewohnt zu föhnen, zu kämmen und zu stylen. Dabei wird ein gesundes, geschmeidiges und glänzendes Erscheinungsbild der Haare erzielt. Gleichzeitig wird die statische Aufladung der Haare verringert, so dass die Handhabung der Haare beim Styling verbessert wird. Die Haarstruktur wird regeneriert, insbesondere die äußere Schuppenschicht der Haare geglättet, so dass krauses Haar bzw. "Frizz-Hair" geglättet wird und ein gepflegtes Erscheinungsbild resultiert.

Gemäß einer weiteren bevorzugten Ausführungsform kann es auch vorgesehen sein, die erfindungsgemäße Zusammensetzung als Haarkur zu verwenden. Dabei wird das Haaröl bzw. die Pflegekur über einen Zeitraum von einer bis hin zu acht Stunden oder über Nacht einwirken gelassen. Anschließend erfolgt ein Auswaschen des Haaröls mit einem milden Shampoo. Auf dieser Basis kann das Haaröl eine noch intensivere Wirkung entfalten, insbesondere im Hinblick auf einen Übergang der Nährstoffe aus dem Öl in Richtung Haarwurzel bzw. Haarfolikel. Auf diese Weise kann sogar ein durch Nährstoffmangel bedingter Haarausfall durch eine Stärkung der Haarwurzel gelindert werden. Darüber hinaus bewirkt die Verwendung des Haaröls als Haarkur bzw. Pflegekur eine Neutralisierung des pH-Werts der Kopfhaut, so dass einem schnellen Nachfetten der Haare entgegengewirkt wird und darüber hinaus eine trockene Kopfhaut gelindert bzw. mit Feuchtigkeit versorgt werden kann.

Gemäß einer weiteren bevorzugten Ausführungsform kann es im Rahmen der vorliegenden Erfindung auch vorgesehen sein, die erfindungsgemäße Zusammensetzung als Styling-Finish für einen intensiven Glanz und eine Intensivierung der Haarfarbe im Rahmen des Stylings zu verwenden. Dabei wird gleichzeitig auch trockenen Haarspitzen, welche langfristig zu Haarbruch und Spliss führen, vorgebeugt.

Weiterhin kann es auch vorgesehen sein, das erfindungsgemäße Haaröl bzw. die Zusammensetzung nach der vorliegenden Erfindung als pflegenden Zusatz in Haarfarben oder Colorationen zu verwenden. Dabei werden wenige Milliliter des Haaröls, abgestimmt auf Haardichte und Haarstruktur, in die Haarfarbe eingerührt. Wie zuvor ausgeführt, stellen Colorationen, insbesondere Blondieren unter Einsatz von Wasserstoffperoxid, eine hohe Belastung für die Haare dar, da die Haarstruktur für die Aufnahme der Pigmente geöffnet werden muss. In der Folge trocknet das Haar aus, bricht schneller ab und fällt darüber hinaus auch leichter aus. Die mit Colorationen, Blondierungen und Haartönungen häufig einhergehenden unerwünschten Nebeneffekte können mit dem Haaröl signifikant gelindert oder verhindert werden. Dabei ist das Haaröl für alle Haartypen, d.h. feine Haare, dicke Haare, Locken, glatte Haare und krause oder geschädigte Haare, und gleichermaßen für alle Haarfarben, d.h. blond, braun, schwarz und rot, geeignet.

Im Zusammenhang mit der Verwendung des Haaröls hat es sich zudem als vorteilhaft erwiesen, wenn dieses in einer geeigneten Applikationsvorrichtung vorliegt. Als besonders geeignet hat sich eine Applikationsvorrichtung erwiesen, welche in Form eines Behältnisses vorliegt, wobei das Behältnis ein Reservoir mit der vorgenannten Zusammensetzung und eine Dosiervorrichtung aufweist, und wobei die Dosiervorrichtung zur Abgabe der Zusammensetzung ausgebildet ist und eine Tropf- oder Sprüheinheit, insbesondere mit Pump-basiertem Sprühmechanismus, aufweist.

Die Bereitstellung einer geeigneten Applikationsvorrichtung zur Abgabe der flüssigen Zusammensetzung, insbesondere des Haaröls nach der vorliegenden Erfindung, ist dem Fachmann grundsätzlich bekannt, so dass es diesbezüglich keiner weiteren Ausführungen bedarf.

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Darüber hinaus wird an dieser Stelle nochmals hervorgehoben, dass Einzelheiten, welche nur zu einem Erfindungsaspekt ausgeführt wurden, selbstverständlich auch für die übrigen Erfindungsaspekte gelten.

Die folgenden Ausführungsbeispiele dienen lediglich der Veranschaulichung der vorliegenden Erfindung, ohne die vorliegende Erfindung jedoch hierauf zu beschränken.

### Ausführungsbeispiele:

### 1. Bereitstellung einer erfindungsgemäßen Zusammensetzung

Die Herstellung einer Zusammensetzung nach der vorliegenden Erfindung in Form eines Haaröls kann gemäß der nachfolgenden Rezeptur erfolgen:

| **Inhaltsstoff** | **Menge [Vol.-%]** |
|---|---|
| Kokosöl | 40 |
| Olivenöl | 11 |
| Sojaöl | 11 |
| Mandelöl | 11 |
| Walnussöl | 11 |
| Jojobaöl | 11 |
| Emulgator (Fluidlecithin Super) | 2 |
| Konservierungsmittel (Antiranz) | 1 |

Zur Bereitstellung der Zusammensetzung werden die Inhaltsstoffe gemäß der obigen Rezeptur gemischt und unter Rühren homogenisiert. Die Herstellung erfolgt mit dem Fachmann grundsätzlich bekannten Verfahren, so dass bezüglich der Herstellung als solcher keine weiteren Ausführungen notwendig sind.

Auf Basis der vorgenannten Rezeptur wird ein Haaröl erhalten, welches eine gegenüber herkömmlichen Ölen verringerte Viskosität, d.h. eine deutlich erhöhte Dünnflüssigkeit, und einen angenehmen, insbesondere natürlichen Duft nach Kokos, Mandel und Jojoba besitzt.

### 2. Wirksamkeit der erfindungsgemäßen Zusammensetzungen

Die Zusammensetzung gemäß der obigen Rezeptur besitzt eine hervorragende Wirksamkeit bei der Behandlung keratinischer Fasern, insbesondere menschlicher Kopfhaare. Das Haaröl spendet dem Haar Feuchtigkeit, so dass es deutlich weniger spröde wirkt und gleichzeitig Haarbruch und Spliss deutlich reduziert werden können. Darüber hinaus wird die Kämmbarkeit, insbesondere nach dem Waschen, deutlich verbessert. Auch wird die Handhabung der Haare, insbesondere beim Frisieren und Stylen, deutlich verbessert, da die Geschmeidigkeit der Haare erhöht und die elektrostatische Aufladung reduziert wird. Schließlich tritt trotz der glättenden Wirkung und Erhöhung der Geschmeidigkeit kein unerwünschter "Build-up-Effekt" auf, wie er häufig bei Verwendung von Pflegeprodukten auf Basis von Silikonen, Silikonölen oder quartären Ammoniumverbindungen beobachtet wird.

Um die Wirksamkeit des erfindungsgemäßen Haaröls weiterführend zu untersuchen, wurde das Haaröl von 100 Probandinnen im Alter von 18 bis 35 Jahren über einen Zeitraum von einem Monat dreimal wöchentlich zur Haarpflege verwendet. Dazu wurden nach der Haarwäsche einige Tropfen des Haaröls zur Pflege in die Haare einmassiert. Anschließend wurde das Haar wie gewohnt gestylt. Bei 30 der Probandinnen wurde zudem eine Blondierung mit peroxidhaltigem Haarfärbemittel vorgenommen, wobei dem Haarfärbemittel einige Tropfen des Haaröls zum Schutz der Haare vor Schädigungen zugesetzt wurde.

Nach einmonatiger Anwendung wurden die Probandinnen angehalten, das Haaröl im Hinblick auf die nachfolgend aufgeführten Wirkungen bzw. Eigenschaften zu beurteilen:
∘ Geschmeidigkeit der Haare
∘ feuchtigkeitsspendende Wirkung
∘ Vorbeugung von Spliss
∘ Anti-Schuppen-Wirkung
∘ Vorbeugung von fettiger Kopfhaut bzw. eines schnellen Nachfettens
∘ Hitzeschutz
∘ antistatische Effekte
∘ Glanz
∘ Kämmbarkeit
∘ Reparatur der Spitzen
∘ Regenerierung trockener Haare
∘ Pflegewirkung
∘ Anti-Haarausfall-Wirkung (bei Haarausfall aufgrund von Nährstoffmangel im Bereich der Haarwurzel)
∘ Kräftigung der Haare
∘ Verbesserung des Volumens
∘ Regenerierung der Haarstruktur (z.B. von Locken)
∘ Vorbeugung von trockener Kopfhaut
∘ Unterstützung der (natürlichen) Haarfarbe
∘ Schutz der Haarstruktur bei Blondierungen (durch Zusatz in Haarfärbemittel)

Im Rahmen der Beurteilung wurde von den Probandinnen angegeben, ob mit dem erfindungsgemäßen Haaröl eine sehr gute (d.h. sehr überdurchschnittliche), eine gute (d.h. eine überdurchschnittliche), eine mittlere (d.h. durchschnittliche) oder eine schlechte (d.h. eine unterdurchschnittliche) Wirkung im Hinblick auf die obigen Kriterien im Vergleich zu handelsüblichen Haarpflegeprodukten bzw. Haarkuren erzielt wurde. Die diesbezüglich erhaltenen durchschnittlichen Ergebnisse lassen sich der nachfolgenden Tabelle entnehmen:

| **Kriterium** | **Sehr gut** | **Gut** | **Mittel** | **Schlecht** |
|---|---|---|---|---|
| Geschmeidigkeit | X | | | |
| Feuchtigkeitsspendende Wirkung | X | | | |
| Vorbeugung von Spliss | X | | | |
| Anti-Schuppen-Wirku ng | X | | | |
| Vorbeugung von fettiger Kopfhaut / Nachfetten | X | | | |
| Hitzeschutz | X | | | |
| antistatische Effekte | | X | | |
| Glanz | X | | | |
| Kämmbarkeit | X | | | |
| Reparatur der Spitzen | X | | | |
| Regenerierung trockener Haare | X | | | |
| Pflegewirkung | X | | | |
| Anti-Haarausfall | | X | | |
| Kräftigung der Haare | X | | | |
| Verbesserung des Volumens | X | | | |
| Regenerierung der Haarstruktur | X | | | |
| Vorbeugung von trockener Kopfhaut | X | | | |
| Unterstützung der Haarfarbe | X | | | |
| Schutz der Haarstruktur bei Blondierungen | X | | | |

Aus den in der Tabelle enthaltenen Ergebnissen ist ersichtlich, dass mit dem erfindungsgemäßen Haaröl im Hinblick auf fast alle der untersuchten Kriterien eine weit über dem Durchschnitt liegende Wirkung bei der Haarpflege erzielt wird. Auch im Hinblick auf die Vorbeugung von Haarausfall, welcher im Zusammenhang mit Nährstoffmangel im Bereich der Haarwurzel steht, und antistatische Effekte wird ebenfalls noch eine über dem Durchschnitt liegende Wirkung erzielt.

Insgesamt wird das Haar auf Basis des erfindungsgemäßen Haaröls vor Schäden geschützt und gleichzeitig intensiv gepflegt bzw. repariert. Ohne sich hierbei auf diese Theorie beschränken zu wollen, wird davon ausgegangen, dass das Öl einerseits in die Haarstruktur eindringt und Schäden bzw. Lücken in der Haarstruktur auffüllt. Darüber hinaus wird auch ein leichter, vor Austrocknung schützender Film um die Haarstruktur gelegt, ohne jedoch das Haar zu beschweren. Darüber hinaus - ebenfalls ohne sich auf diese Theorie beschränken zu wollen - ist der Film jedoch im Gegensatz zu den aus dem Stand der Technik bekannten Silikonen und quartären Ammoniumverbindungen auswaschbar, so dass der bei zahlreichen aus dem Stand der Technik bekannten Produkten beobachtete Build-up-Effekt vermieden wird.

Andererseits kann das Öl auch in die Haarwurzel eindringen, so dass diese weiterführend mit Nährstoffen versorgt wird. Auf dieser Basis kann insbesondere einem durch Nährstoffmangel bedingten Haarausfall, wie er oftmals in Folge von Haarbehandlungen bzw. Haarfärbungen resultiert, vorgebeugt werden.

Dem Haar wird nach Verwendung des Haaröl eine sofortige, langanhaltende Geschmeidigkeit, eine gute Kämmbarkeit und Glanz verliehen. Gleichzeitig wird das Haar vor Hitze geschützt, was insbesondere im Hinblick auf Stylingtechniken unter Einsatz von Hitze, wie z.B. Lockenstäben, vorteilhaft ist.

Neben der hervorragenden Wirksamkeit als Haarpflege bzw. Haarkur eignet sich das Haaröl, wie aus den in der Tabelle enthaltenen Ergebnissen ebenfalls hervorgeht, auch als Pflegezusatz in Haarfärbungen, insbesondere Blondierungen. Die im Allgemeinen bei Haarfärbungen, im besonderen für den Fall, dass Peroxid zur Öffnung der Haarstruktur zum Einsatz kommt, auftretenden Schäden können durch eine Beimischung des Haaröls in das Haarfärbemittel signifikant reduziert werden.

Aufgrund der verschiedenen positiven Eigenschaften ist das erfindungsgemäße Haaröl äußerst vielseitig einsetzbar, nämlich insbesondere als Haarpflege, als Haarkur, als Hitzeschutz und darüber hinaus auch als Zusatz in Haarfärbemitteln.

## Patentansprüche

1. Kosmetische Zusammensetzung, insbesondere Haaröl, wobei die Zusammensetzung als Inhaltsstoffe
Kokosöl in einer Menge im Bereich von 20 bis 70 Vol.-%, bezogen auf die Zusammensetzung,
Olivenöl in einer Menge im Bereich von 1 bis 20 Vol.-%, bezogen auf die Zusammensetzung,
Sojaöl in einer Menge im Bereich von 1 bis 20 Vol.-%, bezogen auf die Zusammensetzung,
Mandelöl in einer Menge im Bereich von 1 bis 20 Vol.-%, bezogen auf die Zusammensetzung,
Walnussöl in einer Menge im Bereich von 1 bis 20 Vol.-%, bezogen auf die Zusammensetzung und
Jojobaöl in einer Menge im Bereich von 1 bis 20 Vol.-%, bezogen auf die Zusammensetzung enthält, und
wobei die Zusammensetzung frei ist von Polyorganosiloxanen, Polysiloxanen, Parabenen, Phthalsäuren sowie deren Salzen und/oder Estern, Quaternium, Polyquaternium, schaumbildenden Sulfaten und zugesetzten Parfumstoffen und Duftstoffen.

2. Zusammensetzung nach Anspruch 1,
wobei die Zusammensetzung das Kokosöl in einer Menge im Bereich von 30 bis 50 Vol.-%, bevorzugt 35 bis 45 Vol.-%, bezogen auf die Zusammensetzung, enthält; und/oder wobei die Zusammensetzung das Olivenöl in einer Menge im Bereich von 5 bis 15 Vol.-%, besonders bevorzugt 8 bis 12 Vol.-%, bezogen auf die Zusammensetzung, enthält.

3. Zusammensetzung nach Anspruch 1 oder 2,
wobei die Zusammensetzung das Sojaöl in einer Menge im Bereich von 5 bis 15 Vol.-%, besonders bevorzugt 8 bis 12 Vol.-%, bezogen auf die Zusammensetzung, enthält; und/oder
wobei die Zusammensetzung das Mandelöl in einer Menge im Bereich von 5 bis 15 Vol.-%, besonders bevorzugt 8 bis 12 Vol.-%, bezogen auf die Zusammensetzung, enthält.

4. Zusammensetzung nach einem der vorangehenden Ansprüche,
wobei die Zusammensetzung das Walnussöl in einer Menge im Bereich von 5 bis 15 Vol.-%, besonders bevorzugt 8 bis 12 Vol.-%, bezogen auf die Zusammensetzung, enthält; und/oder
wobei die Zusammensetzung das Jojobaöl in einer Menge im Bereich von 5 bis 15 Vol.-%, besonders bevorzugt 8 bis 12 Vol.-%, bezogen auf die Zusammensetzung, enthält.

5. Zusammensetzung nach einem der vorangehenden Ansprüche, wobei die Zusammensetzung Olivenöl, Sojaöl, Mandelöl, Walnussöl und Jojobaöl jeweils zu zumindest im Wesentlichen gleichen Volumenanteilen enthält und/oder wobei die Zusammensetzung Olivenöl, Sojaöl, Mandelöl, Walnussöl und Jojobaöl in einem volumenbezogenen Verhältnis von Olivenöl : Sojaöl: Mandelöl : Walnussöl : Jojobaöl von zumindest im Wesentlichen 1 : 1 : 1 : 1 : 1 enthält.

6. Zusammensetzung nach einem der vorangehenden Ansprüche, wobei die Zusammensetzung das Kokosöl und die weiteren Öle [Olivenöl, Sojaöl, Mandelöl, Walnussöl und Jojobaöl] in einem volumenbezogenen Verhältnis von Kokosöl zu den weiteren Ölen [Olivenöl, Sojaöl, Mandelöl, Walnussöl und Jojobaöl] im Bereich von 1 : 10 bis 10 : 1, insbesondere 1 : 5 bis 5 : 1, vorzugsweise 1 : 3 bis 3 : 1, bevorzugt 1 : 2 bis 1 : 1, enthält.

7. Zusammensetzung nach einem der vorangehenden Ansprüche, wobei die Zusammensetzung als weiteren Inhaltsstoff mindestens einen Emulgator enthält, insbesondere in einer Menge im Bereich von 0,01 bis 10 Vol.-%, insbesondere 0,1 bis 8 Vol.-%, vorzugsweise 1 bis 5 Vol.-%, bezogen auf die Zusammensetzung.

8. Zusammensetzung nach Anspruch 7,
wobei der Emulgator Phospholipide, insbesondere Phosphatidylcholine (Lecithine), enthält, insbesondere in einer Menge im Bereich von 20 bis 90 Vol.-%, vorzugsweise 30 bis 80 Vol.-%, bevorzugt 40 bis 70 Vol.-%, bezogen auf den Emulgator; und/oder
wobei der Emulgator mindestens ein pflanzliches Öl, insbesondere Distelöl und/oder Sonnenblumenöl, vorzugsweise Distelöl, enthält, insbesondere wobei der Emulgator das mindestens eine pflanzliche Öl in einer Menge im Bereich von 20 bis 90 Vol.-%, vorzugweise 30 bis 80 Vol.-%, bevorzugt 40 bis 70 Vol.-%, bezogen auf den Emulgator, enthält; und/oder
wobei der Emulgator weitere kosmetische Hilfsstoffe und/oder Additive enthält, insbesondere ausgewählt aus der Gruppe von (i) Glycerin; (ii) mittelkettigen Triglyceriden (MCT-Fette), insbesondere Capronsäuren, Caprylsäuren, Caprinsäuren, Laurinsäuren sowie deren kosmetisch verträglichen Salzen, vorzugsweise Caprylsäuren und/oder Caprinsäuren sowie deren kosmetisch verträglichen Salzen; (iii) Alkoholen, insbesondere Ethanol; (iv) Glycinen und/oder deren kosmetisch verträglichen Salzen, insbesondere Glycerinstearinsäure und/oder deren kosmetisch verträgliche Salzen; (v) Palmitinsäuren und/oder deren kosmetisch verträglichen Salzen und/oder Estern, insbesondere Palmitinsäureascorbylester.

9. Zusammensetzung nach einem der vorangehenden Ansprüche, wobei die Zusammensetzung als weiteren Inhaltsstoff Proteine und/oder Hydrolysat von Proteinen enthält, insbesondere in einer Menge im Bereich von 0,001 bis 5 Gew.-%, insbesondere 0,01 bis 4 Gew.-%, vorzugsweise 0,1 bis 3 Gew.-%, bezogen auf die Zusammensetzung.

10. Zusammensetzung nach Anspruch 9,
wobei die Proteine und/oder die Hydrolysate von Proteinen ausgewählt sind aus der Gruppe von Proteinen tierischen Ursprungs sowie Hydrolysaten davon, insbesondere Keratin, Elastinen, Kollagenen und/oder Seidenproteinen sowie Hydrolysaten davon, und/oder
wobei die Proteine und/oder die Hydrolysate von Proteinen ausgewählt sind aus der Gruppe von Proteinen pflanzlichen Ursprungs sowie Hydrolysaten davon, insbesondere Sojaproteinen, Weizenproteinen und/oder Haferproteinen sowie Hydrolysaten davon, bevorzugt Weizenproteinen sowie Hydrolysaten davon.

11. Zusammensetzung nach einem der vorangehenden Ansprüche, wobei die Zusammensetzung mindestens ein Konservierungsmittel enthält, insbesondere in einer Menge im Bereich von 0,01 bis 10 Vol.-%, insbesondere 0,05 bis 8 Vol.-%, vorzugsweise 0,1 bis 5 Vol.-%, bezogen auf die Zusammensetzung.

12. Zusammensetzung nach Anspruch 11, wobei das Konservierungsmittel Antioxidantien enthält, insbesondere Vitamin C (Ascorbinsäure und/oder deren Salze), Vitamin E (Tocopherole und/oder Tocotrienole) und/oder Citronensäure und/oder deren Salze.

13. Zusammensetzung nach einem der vorangehenden Ansprüche, wobei die Zusammensetzung mindestens einen weiteren kosmetisch verträglichen Hilfsstoff und/oder mindestens ein weiteres kosmetisch verträgliches Additiv enthält, insbesondere ausgewählt aus der Gruppe von (i) Stabilisatoren; (ii) Filmbildnern; (iii) Rheologiemodifikatoren; (iv) Duftstoffen und/oder Parfumstoffen; und/oder (v) Farbstoffen und/oder Pigmenten.

14. Verwendung einer Zusammensetzung, insbesondere des Haaröls, nach einem der vorangehenden Ansprüche im Bereich der Kosmetik, insbesondere zur Behandlung und/oder Pflege keratinischer Fasern, vorzugsweise menschlicher Haare.

## Claims

1. Cosmetic composition, in particular hair oil, the composition comprising as ingredients
coconut oil in an amount ranging from 20 to 70 % by volume, based on the composition,
olive oil in an amount ranging from 1 to 20 % by volume, based on the composition, soybean oil in an amount ranging from 1 to 20 % by volume, based on the composition,
almond oil in an amount ranging from 1 to 20 % by volume, based on the composition, walnut oil in an amount ranging from 1 to 20 % by volume, based on the composition, and
jojoba oil in an amount ranging from 1 to 20 % by volume, based on the composition, and
wherein the composition is free of polyorganosiloxanes, polysiloxanes, parabens, phthalic acids and their salts and/or esters, quaternium, polyquaternium, foam-forming sulfates and added perfumes and fragrances.

2. The composition according to claim 1,
wherein the composition comprises coconut oil in an amount ranging from 30 to 50 % by volume, preferably 35 to 45 % by volume, based on the composition; and/or wherein the composition comprises olive oil in an amount ranging from 5 to 15 % by volume, more preferably 8 to 12 % by volume, based on the composition.

3. A composition according to claim 1 or 2,
wherein the composition comprises soybean oil in an amount ranging from 5 to 15 % by volume, more preferably from 8 to 12 % by volume, based on the composition; and/or
wherein the composition comprises almond oil in an amount ranging from 5 to 15 % by volume, more preferably 8 to 12 % by volume, based on the composition.

4. A composition according to any one of the preceding claims,
wherein the composition comprises walnut oil in an amount ranging from 5 to 15 % by volume, more preferably from 8 to 12 % by volume, based on the composition; and/or
wherein the composition comprises jojoba oil in an amount ranging from 5 to 15 % by volume, more preferably 8 to 12 % by volume, based on the composition.

5. A composition according to any one of the preceding claims, wherein the composition comprises olive oil, soybean oil, almond oil, walnut oil and jojoba oil each in at least substantially equal volume proportions and/or wherein the composition comprises olive oil, soybean oil, almond oil, walnut oil and jojoba oil in a volume ratio of olive oil : soybean oil : almond oil : walnut oil : jojoba oil of at least substantially 1 : 1 : 1 : 1 : 1.

6. A composition according to any one of the preceding claims, wherein the composition comprises coconut oil and the further oils [olive oil, soybean oil, almond oil, walnut oil and jojoba oil] in a volume ratio of coconut oil to the further oils [olive oil, soybean oil, almond oil, walnut oil and jojoba oil] in the range of 1 : 10 to 10 : 1, in particular 1 : 5 to 5 : 1, preferably 1 : 3 to 3 : 1, preferably 1 : 2 to 1 : 1.

7. Composition according to any one of the preceding claims, wherein the composition comprises as further ingredient at least one emulsifier, in particular in an amount ranging from 0.01 to 10 % by volume, in particular from 0.1 to 8 % by volume, preferably from 1 to 5 % by volume, based on the composition.

8. Composition according to claim 7,
wherein the emulsifier contains phospholipids, in particular phosphatidylcholines (lecithins), in particular in an amount in the range from 20 to 90 % by volume, preferably 30 to 80 % by volume, preferably 40 to 70 % by volume, based on the emulsifier; and/or
wherein the emulsifier contains at least one vegetable oil, in particular safflower oil and/or sunflower oil, preferably safflower oil, in particular wherein the emulsifier contains the at least one vegetable oil in an amount in the range from 20 to 90 % by volume, preferably 30 to 80 % by volume, preferably 40 to 70 % by volume, based on the emulsifier; and/or
the emulsifier containing further cosmetic auxiliaries and/or additives, in particular selected from the group consisting of (i) glycerol; (ii) medium-chain triglycerides (MCT fats), in particular caproic acids, caprylic acids, capric acids, lauric acids and their cosmetically acceptable salts, preferably caprylic acids and/or capric acids and their cosmetically acceptable salts; (iii) alcohols, in particular ethanol; (iv) glycines and/or cosmetically acceptable salts thereof, in particular glycerostearic acid and/or cosmetically acceptable salts thereof; (v) palmitic acids and/or cosmetically acceptable salts thereof and/or esters, in particular palmitic acid ascorbyl esters.

9. Composition according to any one of the preceding claims, wherein the composition contains as further ingredient proteins and/or hydrolysate of proteins, in particular in an amount in the range from 0.001 to 5 % by weight, in particular 0.01 to 4 % by weight, preferably 0.1 to 3 % by weight, based on the composition.

10. Composition according to claim 9,
wherein the proteins and/or the hydrolysates of proteins are selected from the group of proteins of animal origin as well as hydrolysates thereof, in particular keratin, elastins, collagens and/or silk proteins as well as hydrolysates thereof, and/or
wherein the proteins and/or the hydrolysates of proteins are selected from the group of proteins of vegetable origin as well as hydrolysates thereof, in particular soy proteins, wheat proteins and/or oat proteins as well as hydrolysates thereof, preferably wheat proteins as well as hydrolysates thereof.

11. Composition according to any one of the preceding claims, wherein the composition comprises at least one preservative, in particular in an amount ranging from 0.01 to 10 % by volume, in particular from 0.05 to 8 % by volume, preferably from 0.1 to 5 % by volume, based on the composition.

12. Composition according to claim 11, wherein the preservative contains antioxidants, in particular vitamin C (ascorbic acid and/or salts thereof), vitamin E (tocopherols and/or tocotrienols) and/or citric acid and/or salts thereof.

13. Composition according to any one of the preceding claims, wherein the composition comprises at least one further cosmetically acceptable excipient and/or at least one further cosmetically acceptable additive, in particular selected from the group of (i) stabilizers; (ii) film formers; (iii) rheology modifiers; (iv) fragrances and/or perfumes; and/or (v) dyes and/or pigments.

14. Use of a composition, in particular the hair oil, according to one of the preceding claims in the field of cosmetics, in particular for the treatment and/or care of keratinous fibers, preferably human hair.

## Revendications

1. Composition cosmétique, en particulier huile capillaire, la composition comprenant comme ingrédients
l'huile de coco en quantité allant de 20 à 70 % en volume, par rapport à la composition, l'huile d'olive dans une quantité allant de 1 à 20 % en volume, par rapport à la composition,
l'huile de soja en quantité allant de 1 à 20 % en volume, par rapport à la composition, l'huile d'amande en quantité allant de 1 à 20 % en volume, par rapport à la composition,
de l'huile de noix en une quantité allant de 1 à 20 % en volume, par rapport à la composition, et
de l'huile de jojoba en une quantité allant de 1 à 20 % en volume, par rapport à la composition, et
dans laquelle la composition est exempte de polyorganosiloxanes, polysiloxanes, parabènes, acides phtaliques et leurs sels et/ou esters, quaternium, polyquaternium, sulfates moussants et de parfums et fragrances ajoutés.

2. Une composition selon la revendication 1,
dans laquelle la composition comprend l'huile de coco en une quantité allant de 30 à 50 % en volume, de préférence de 35 à 45 % en volume, par rapport à la composition; et/ou
dans laquelle la composition comprend l'huile d'olive dans une quantité allant de 5 à 15 % en volume, de préférence de 8 à 12 % en volume, par rapport à la composition.

3. Composition selon la revendication 1 ou 2,
dans laquelle la composition comprend l'huile de soja en une quantité allant de 5 à 15 % en volume, de préférence de 8 à 12 % en volume, par rapport à la composition; et/ou
dans laquelle la composition comprend l'huile d'amande en une quantité allant de 5 à 15 % en volume, de préférence de 8 à 12 % en volume, par rapport à la composition.

4. Une composition selon l'une des revendications précédentes,
dans laquelle la composition comprend l'huile de noix en une quantité allant de 5 à 15 % en volume, de préférence de 8 à 12 % en volume, par rapport à la composition; et/ou
dans laquelle la composition comprend l'huile de jojoba en une quantité allant de 5 à 15 % en volume, de préférence de 8 à 12 % en volume, par rapport à la composition.

5. Composition selon l'une quelconque des revendications précédentes, dans laquelle la composition comprend de l'huile d'olive, de l'huile de soja, de l'huile d'amande, de l'huile de noix et de l'huile de jojoba, chacune dans des proportions en volume au moins sensiblement égales et/ou dans laquelle la composition comprend de l'huile d'olive, de l'huile de soja, de l'huile d'amande, de l'huile de noix et de l'huile de jojoba dans un rapport en volume huile d'olive : huile de soja : huile d'amande : huile de noix : huile de jojoba d'au moins sensiblement 1 : 1 : 1 : 1 : 1.

6. Composition selon l'une quelconque des revendications précédentes, dans laquelle la composition comprend l'huile de coco et les autres huiles [huile d'olive, huile de soja, huile d'amande, huile de noix et huile de jojoba] dans un rapport en volume de l'huile de coco aux autres huiles [huile d'olive, huile de soja, huile d'amande, huile de noix et huile de jojoba] dans la plage de 1:10 à 10:1, en particulier 1:5 à 5:1, de préférence 1:3 à 3:1, de préférence 1:2 à 1:1.

7. Composition selon l'une quelconque des revendications précédentes, dans laquelle la composition comprend comme autre ingrédient au moins un émulsifiant, en particulier en une quantité allant de 0,01 à 10 % en volume, en particulier de 0,1 à 8 % en volume, de préférence de 1 à 5 % en volume, par rapport à la composition.

8. Composition selon la revendication 7,
dans lequel l'émulsifiant contient des phospholipides, en particulier des phosphatidylcholines (lécithines), notamment en une quantité comprise entre 20 et 90 % en volume, de préférence entre 30 et 80 % en volume, de préférence entre 40 et 70 % en volume, par rapport à l'émulsifiant; et/ou
dans lequel l'émulsifiant contient au moins une huile végétale, en particulier de l'huile de carthame et/ou de l'huile de tournesol, de préférence de l'huile de carthame, en particulier dans lequel l'émulsifiant contient la au moins une huile végétale en une quantité comprise dans la plage de 20 à 90 % en volume, de préférence de 30 à 80 % en volume, de préférence de 40 à 70 % en volume, par rapport à l'émulsifiant; et/ou
l'émulsifiant contenant d'autres adjuvants et/ou additifs cosmétiques, en particulier choisis dans le groupe constitué par (i) le glycérol; (ii) les triglycérides à chaîne moyenne (graisses MCT), en particulier les acides caproïques, les acides capryliques, les acides capriques, les acides lauriques et leurs sels cosmétiquement acceptables, de préférence les acides capryliques et/ou les acides capriques et leurs sels cosmétiquement acceptables; (iii) les alcools, en particulier l'éthanol; (iv) les glycines et/ou leurs sels cosmétiquement acceptables, en particulier l'acide glycérostéarique et/ou ses sels cosmétiquement acceptables; (v) les acides palmitiques et/ou leurs sels cosmétiquement acceptables et/ou leurs esters, en particulier les esters ascorbyliques de l'acide palmitique.

9. Composition selon l'une quelconque des revendications précédentes, dans laquelle la composition comprend comme autre ingrédient des protéines et/ou un hydrolysat de protéines, en particulier en une quantité allant de 0,001 à 5 % en poids, en particulier de 0,01 à 4 % en poids, de préférence de 0,1 à 3 % en poids, par rapport à la composition.

10. Composition selon la revendication 9,
dans laquelle les protéines et/ou les hydrolysats de protéines sont choisis dans le groupe des protéines d'origine animale ainsi que de leurs hydrolysats, en particulier la kératine, les élastines, les collagènes et/ou les protéines de soie ainsi que leurs hydrolysats, et/ou
dans laquelle les protéines et/ou les hydrolysats de protéines sont choisis dans le groupe des protéines d'origine végétale ainsi que leurs hydrolysats, en particulier les protéines de soja, les protéines de blé et/ou les protéines d'avoine ainsi que leurs hydrolysats, de préférence les protéines de blé ainsi que leurs hydrolysats.

11. Composition selon l'une quelconque des revendications précédentes, **caractérisée en ce que** la composition comprend au moins un conservateur, notamment en une quantité allant de 0,01 à 10 % en volume, notamment de 0,05 à 8 % en volume, de préférence de 0,1 à 5 % en volume, par rapport à la composition.

12. Composition selon la revendication 11, dans laquelle le conservateur comprend des antioxydants, en particulier de la vitamine C (acide ascorbique et/ou ses sels), de la vitamine E (tocophérols et/ou tocotriénols) et/ou de l'acide citrique et/ou ses sels.

13. Composition selon l'une quelconque des revendications précédentes, dans laquelle la composition comprend au moins un autre excipient cosmétiquement acceptable et/ou au moins un autre additif cosmétiquement acceptable, en particulier choisi dans le groupe constitué par (i) des stabilisants; (ii) des agents filmogènes; (iii) des modificateurs de rhéologie; (iv) des parfums et/ou des fragrances; et/ou (v) des colorants et/ou des pigments.

14. Utilisation d'une composition, en particulier d'une huile capillaire, selon l'une des revendications précédentes dans le domaine des cosmétiques, en particulier pour traiter et/ou soigner les fibres kératiniques, de préférence les cheveux humains.
